Europäisches Patentamt

European Patent Office

Office européen des brevets

(11) Publication number: **0 222 710**
**A2**

(12) **EUROPEAN PATENT APPLICATION**

(21) Application number: 86830313.2

(22) Date of filing: 29.10.86

(51) Int. Cl.⁴: **F 28 F 21/06**
**A 61 M 1/36**

(30) Priority: 13.11.85 IT 1740885

(43) Date of publication of application:
20.05.87 Bulletin 87/21

(84) Designated Contracting States:
AT BE CH DE ES FR GB GR IT LI LU NL SE

(71) Applicant: SORIN BIOMEDICA S.p.A.
Strada per Crescentino 31
I-13040 Saluggia (Vercelli) (IT)

(72) Inventor: Borglone, Teresa
Viale Matteotti 20
I-57025 Piombino(Livorno) (IT)

(74) Representative: Bosotti, Luciano et al
c/o Jacobacci-Casetta & Perani S.p.A. Via Alfieri, 17
I-10121 Torino (IT)

(54) **Heat exchanger for fluids.**

(57) A heat exchanger for fluids, which is arranged to be inserted in apparatus having a limited period of use, is constituted by a plurality of tubes (2) of synthetic resin disposed in parallel in a container (1) with a constant cross-section, the physical and geometrical characteristics of the whole being determined by the conditions of use of the exchanger itself.

FIG. 1

EP 0 222 710 A2

**Description**

Heat exchanger for fluids.

The present invention relates to heat exchangers for fluids for insertion in apparatus having a limited period of use, of which a typical example is a blood oxygenator such as used in cardiac surgery. Such apparatus is normally used for a single operation and then thrown away; its use can therefore be counted in hours.

It is known that, during such operations, the temperature of the patient's blood must be varied: a heat exchanger is thus always inserted in the oxygenator for heating or cooling the blood as necessary.

The heat exchangers usually inserted in oxygenators are based on the conventional types known in the art or differ only slightly from them: they are usually metal plates, finned tubes, straight or coiled, smooth or ribbed tubes,etc., which are generally made from a material with a good heat conductivity, that is, metal.

In apparatus used for short periods of time and then thrown away, metal heat exchangers create a not-insubstantial waste in that a relatively expensive component which is mechanically still sound is discarded.

There have been some proposals for the use of plastics materials (PVC) instead of metal but these have not been found to be practical in use since, as could be foreseen, the results of the heat exchange were quite unsatisfactory in that the structure and dimensions of the metal heat exchangers were left unchanged.

The adoption of cheaper metals than stainless steel, for example aluminium, requires a treatment which is almost always anodising: this constitute a cost which is added to that of the metal, limiting the economic advantages of the substitution.

In avoidance of the disadvantages explained above, the heat exchanger of the invention, which is simple to manufacture, is manageable in use and has considerable economic advantages, as well as ensuring the same results as metal exchangers.

The main subject of the present invention is a heat exchanger arranged to be inserted in apparatus having a limited period of use and constituted by a plurality of tubes of synthetic resin disposed in parallel in a container with a constant cross-section over its entire length, the mechanical characteristics of the resin and the dimensions and number of tubes being determined, for a given heat exchange capacity, by the flow characteristics of the fluids in play and the temperature range within which the heat exchange occurs.

These and other characteristics of the invention will become clear from the following description of a preferred embodiment thereof, taken in combination with the appended drawings, in which:

Figure 1 is a diagrammatic view of the heat exchanger according to the invention with a part of its outer wall removed;

Figure 2 is a diagrammatic representation, in longitudinal median section, of one example of use of the exchanger of Figure 1 in blood oxygenators.

For a better understanding of the scope of the invention, it is necessary to put brief theoretical premise which clarifies the underlying physical principles.

In a heat exchanger, a first fluid enters at a temperature $T_{1i}$ and a second fluid enters at a temperature $T_{2i}$; the internal configuration favours the transfer of heat between the two fluids which leave at respective temperatures $T_{1u}$ and $T_{2u}$. Generally, the efficiency of exchange is characterised by a yield factor Q defined by:

$$Q = \frac{T_{1i} - T_{1u}}{T_{1i} - T_{2i}}$$

where T indicates the temperature of the fluid, the indices 1 and 2 relate to the fluids, and the indices i and u relate to the inlet and outlet, as indicated above.

It is well known from the theory of heat exchange between fluids separated by a wall that the yield Q depends on numerous factors,which are essentially:

a) the conditions of counterflow or concurrent flow of the two fluids;
b) the value of the flows $F_1$ and $F_2$;
c) the thickness s of the wall separating the two flows through which the heat exchange occurs;
d) the surface area A of the wall;
e) the thermal conductivity k of the material of which the wall is made;
f) the heat transmission factors $h_1$ and $h_2$ between the two fluids 1 and 2 and the separating wall.

These factors come together to determine the yield Q as follows:

$$Q = F(F_1, F_2, \frac{Ks}{A}, h_1, h_2) \quad (1)$$

where F is a function which depends on the factor defined in a).

The various factors from b) to f) are in part calculable from physical/geometrical considerations and in part obtainable by experiment, particularly when, as in the case in question, the configuration departs considerably from the ideal, simple one which can be treated theoretically.

It is known, both from experimentation and indirect calculation, that the transfer of heat from the wall to the fluid and vice versa is considerably more important than the transfer of heat through the wall. Precise calculations on conventional exchangers of stainless steel, taking account solely of the transfer of heat through the wall, with $s = 0.2mm$; $A = 0.15m^2$; $k = 0.037cal/sec\ cm°C$; a heating liquid flow of 10 l/min, and a heated liquid flow of 5 l/min, have resulted in a thermal yield factor $Q_1$ close to 1, while experiments carried out, where obviously the wall/fluid heat transfer factor also acted, have given an effective yield factor $Q_2$ of about 0.5.

The immediate deduction is that, in the case in which certain temperature and flow conditions intervene, fully verified in the oxygenators mentioned above, the factor k does not have a primary percentage effect on the thermal yield factor Q; consequently, it is possible to compensate for even a very considerable reduction in the factor k only by suitable modification of any of the other factors which operate in formula (1).

The factors which can be varied most obviously are those of the geometry of the entire unit, such as the thickness of the wall through which the heat exchange occurs and the heat-exchange surface area.

Such variations must naturally be designed within the limits fixed by the mechanical characteristics which the heat exchanger must have.

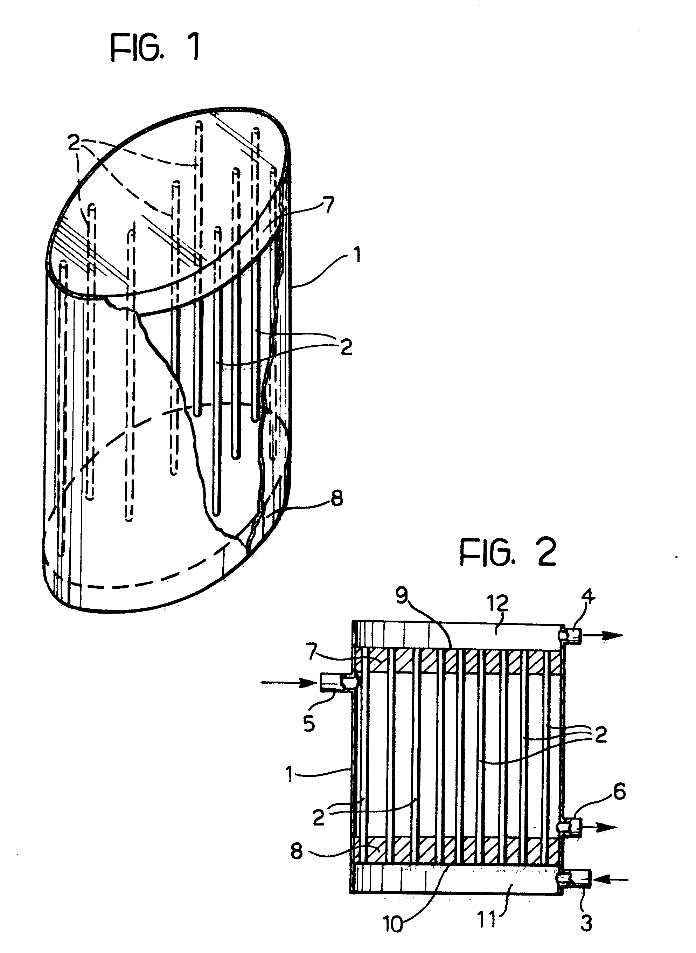

The conditions explained above are fully satisfied by the exchanger of the present invention, shown diagrammatically in Figure 1. In this, a container of plastics material, such as polycarbonate, polyethylene terephthalate, polysulphone or like material, is indicated 1 and has a section of any shape as long as it is constant over its entire length. Within it is arranged a plurality of mutually-parallel tubes 2 disposed longitudinally of the container 1. One of the two fluids, for example, that to be heated (or cooled) flows in these tubes, while the second fluid, in this example the heating (or cooling) fluid, flows in the space defined between the inner surface of the container 1 and the outer surfaces of the walls of the tubes 2. The roles may obviously be reversed, the heating (cooling) fluid being made to flow in the tubes 2 with the heated (cooled) fluid outside them.

The tubes 2 may be of any synthetic resin as long as it has chemical and physical characteristics suitable for the work to be carried out; similarly, the number and length of the tubes depend on the required performance of the heat exchanger. In any case, the dimensions of the tubes 1 and their combination must be determined to take into account both the characteristics of the fluid flows in play and the temperature interval within which the heat exchange occurs. As these conditions vary with variations in the field of application of the hear exchanger, the case of a heat exchanger coupled to a blood oxygenator of any type will be dealt with here in detail, by way of example.

In this application, the temperature of the heated fluid (blood) is regulated between 15 and 37°C; the flow values of the two liquids are normally around 10 l/min for the heating water and 5 l/min for the blood. It will assumed that the heat exchange occurs with the liquids in counterflow; the value of the thermal conductivity of the synthetic material of the tubes will be taken to be an average value for the synthetic resins, which is - in effect - about that of steel multiplied by a factor of $10^{-2}$. Moreover, given that the tubes 2 are in contact with the blood, the synthetic material must be compatible with human blood.

Starting from this basic data, it is found that the material from which the tubes 2 must be made must have a tensile strength of not less than $5kg/mm^2$ and a modulus of elasticity of not less than $200kg/mm^2$; moreover, the material must be such as to enable the inner walls of the tubes to be made with minimal roughness in order to avoid damage to the blood, as is well known to experts in the art.

Among the synthetic resins compatible with blood, various materials have the aforesaid mechanical characteristics: these are polycarbonates, polysulphones, polyethylene terephthalate, and other similar materials. The tubes are made from these materials by extrusion, a method which ensures the smoothness of the inner surface necessitated by the intended use.

These materials have a thermal conductivity k greater than $0.04.10^{-2}\ cal/sec\ cm°C$.

The thickness of the walls should be as small as possible but be such as to ensure, within wide margins, the necessary mechanical characteristics of static and dynamic resistance to the flow of the two fluids and impermeability thereto with temperature.

These conditions being taken into account, the optimum thickness of the walls is between 0.03mm and 0.1mm; the inner diameter of the tubes should be between 0.5mm and 1.2mm, dimensions which also ensure easy movement of the flow within them.

With these values of the diameter and thickness of the walls, an active length of the tubes of between 6 and 12mm has been shown to be totally effective, the active length being understood as the length of the tubes used for the heat exchange.

One factor still remains with which to compensate completely the low thermal conductivity k of the plastics material: this is the area of the total heat-exchange surface. It is found by calculation and confirmed by experiment that an optimum heat-exchange surface must have an area of between 0.3 and $0.8m^2$, which means that there must be between 1500 and 4000 tubes of the dimensions indicated above.

Finally, the combination of the flow data of the two fluids and the heat exchange values have resulted in a determination of the density of the tubes, that is the ratio d between the area of the surface occupied by the tubes 2 and the area of the section of the container 1. It is found from experimental data that the optimum ratio is

$$0.3 < d < 0.8$$

From experimental tests, heat exchangers having the characteristics specified above have produced the heat exchange yield factors Q given below in Table 1.

0 222 710

TABLE 1

| Blood flow | 2 l/min | 4 l/min | 6 l/min |
|---|---|---|---|
| Water/blood in counterflow Q= | 0.698 | 0.617 | 0.575 |
| Water/blood in concurrent flow Q= | 0.657 | 0.603 | 0.561 |

These values correspond extremely well with those normally accepted for metal heat exchangers, which vary between 0.5 and 0.6.

The heat exchanger of the invention, as applied to blood oxygenators, is shown diagrammatically in longitudinal median section in Figure 2. In this, there are indicated 3 and 4 respectively inlet and outlet nozzles for the blood, and 5 and 6 respectively two inlet and outlet nozzles for the water. The terminal parts 7 and 8 of the tubes 2 are fixed by a resin binder (generally polyurethane) and face the surfaces 9 and 10. The method for effecting this fixing is well known in the art: the resin is introduced in the fluid state, centrifuged, and the resin concentrated at the ends is left to solidify.

The blood from the nozzle 3 fills a distribution chamber 11, rises along the tubes 2 the ends of which open onto the surface 10, comes out of these into a connecting chamber 12, and leaves the latter through the nozzle 4.

The water comes in through the nozzle 5, enters the spaces between the tubes 2, descends around the latter to heat or cool the blood which flows in counter-current within the tubes, and leaves through the nozzle 6.

## Claims

1. Heat exchanger for fluids, which is arranged to be inserted in apparatus having a limited period of use, characterised in that it is constituted by a plurality of tubes (2) of synthetic resin disposed in parallel in a container (1) with a constant cross-section over its entire length, the mechanical characteristics of the resin and the dimensions and number of tubes (2) being determined, for a given heat exchange capacity, by the flow characteristics of the fluids in play and the temperature range within which the heat exchange occurs.

2. Heat exchanger as in Claim 1, characterised in that, for its insertion in a blood oxygenator, the tubes (2) have an internal diameter of between 0.5 and 1.2mm, a wall thickness of between 0.03 and 0.1mm, and an active length of between 6 and 12cm.

3. Heat exchanger as in the preceding claims, characterised in that, for its insertion in a blood oxygenator, the synthetic resin have a tensile strength of not less than $5kg/mm^2$, a modulus of elasticity of not less than $200kg/mm^2$, and are compatible with human blood.

4. Heat exchanger as in the preceding claims, characterised in that, for its insertion in a blood oxygenator, the tubes (2) ensure an exchange surface area of not less than $0.3^2$.

5. Heat exchanger as in the preceding claims, characterised in that, for its insertion in a blood oxygenator, the density of the tubes (2) in the container is such that the ratio between the surface area occupied by tubes (2) themselves and the sectional area of the container (1) is greater than 0.3 and less than 0.8.

6. Heat exchanger as in the preceding claims, characterised in that, for its insertion in a blood oxygenator, the material of which the tubes (2) are made is chosen from the group consisting of polycarbonate, polysulphone and polyethyleneterephthalate.

4

FIG. 1

FIG. 2